(19) Europäisches Patentamt
European Patent Office
Office européen des brevets

(11) EP 0 688 558 B1

(12) **FASCICULE DE BREVET EUROPEEN**

(45) Date de publication et mention de la délivrance du brevet: **14.01.1998 Bulletin 1998/03**

(51) Int. Cl.$^6$: **A61K 7/13**

(21) Numéro de dépôt: **95400979.1**

(22) Date de dépôt: **28.04.1995**

(54) **Composition de teinture d'oxydation des fibres kératiniques comprenant la 2-(bêta-hydroxyéthyl) paraphénylènediamine, la 2-méthylrésorcine et la résorcine et procédé de teinture mettant en oeuvre une telle composition**

Zusammensetzung zum oxidativen Färben von Keratinfasern, die 2-(beta-Hydroxyethyl)-p-phenylendiamin, 2-Methylresorcin und Resorcin enthält, und Verfahren zum Färben unter Verwendung dieser Zusammensetzung

Composition for dyeing keratinous fibres comprising 2-(beta-hydroxyethyl)-paraphenylene-diamine, 2-methylresorcin and resorcin

(84) Etats contractants désignés:
**DE ES FR GB IT**

(30) Priorité: **21.06.1994 FR 9407582**

(43) Date de publication de la demande:
**27.12.1995 Bulletin 1995/52**

(73) Titulaire: **L'OREAL**
**75008 Paris (FR)**

(72) Inventeurs:
- **Cotteret, Jean**
  **F-78480 Verneuil sur Seine (FR)**
- **Audousset, Marie-Pascale**
  **F-92600 Asnières (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 400 330**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.15.8/3.4

## Description

La présente invention concerne une composition de teinture d'oxydation des fibres kératiniques et en particulier des fibres kératiniques humaines, comprenant en association, de la 2-β-hydroxyéthyl paraphénylènediamine, de la 2-méthyl résorcine et de la résorcine. Elle concerne également l'utilisation d'une telle composition.

Il est connu de teindre les fibres kératiniques, et en particulier les cheveux humains, avec des compositions de teinture contenant des précurseurs de colorants d'oxydation, en particulier des ortho- ou paraphénylènediamines, des ortho-ou paraaminophénols, généralement appelés "bases d'oxydation", associés à des coupleurs encore appelés modificateurs de coloration, plus particulièrement des métaphénylènediamines, des méta-aminophénols et des métadiphénols, qui permettent de modifier et d'enrichir de reflets les colorations "de fond" obtenues par les produits de condensation des bases d'oxydation.

On recherche, dans le domaine de la teinture capillaire d'oxydation, des précurseurs de colorants d'oxydation et des coupleurs capables d'engendrer, lorsqu'ils sont associés, des nuances ayant une résistance satisfaisante à la lumière, aux lavages, aux intempéries, à la transpiration et aux différents traitements capillaires que peuvent subir les cheveux.

Jusqu'ici, ces nuances ont été obtenues avec des teintures à base de paraphénylènediamine. Toutefois, l'utilisation de la paraphénylènediamine semble, depuis quelque temps, être remise en question notamment pour des raisons toxicologiques.

Aussi, en remplacement de la paraphénylènediamine, on a déjà proposé, dans la demande de brevet WO 80/0014, d'utiliser des dérivés de la paraphénylènediamine monohydroxyalkylés en position 2 sur le noyau benzénique.

Malheureusement, il s'avère que les nuances qui sont obtenues avec des teintures utilisant les dérivés de la paraphénylènediamine monohydroxyalkylés en position 2 sur le noyau benzénique ne présentent pas une résistance suffisante aux shampooings, à la lumière et à la transpiration, notamment lorsque les cheveux à teindre sont des cheveux qui ont été sensibilisés par des traitements capillaires de type chimique, en particulier des traitements de déformation permanente ou de décoloration.

Dans le brevet EP-0 400 330 B1, on a par ailleurs décrit tout particulièrement une association entre de la 2-β-hydroxyéthyl paraphénylènediamine et un coupleur choisi parmi la résorcine, la 4-chloro résorcine, la 2-méthyl résorcine, le 3,4-méthylènedioxy phénol, le 3-amino phénol et la N-(2-hydroxyéthyl) 3,4-méthylènedioxy aniline.

Toutefois, on constate, là encore, que les associations de la 2-β-hydroxyéthyl paraphénylènediamine avec l'un ou l'autre des coupleurs cités ci-dessus engendrent des teintures peu résistantes aux shampooings, à la lumière et à la transpiration, en particulier dans le cas de cheveux sensibilisés par des traitements de déformation permanente ou des traitements de décoloration, ce qui limite fortement l'intérêt pratique de telles associations dans le domaine de la coloration capillaire.

Or, après d'importantes recherches menées sur la question, la Demanderesse vient maintenant de découvrir qu'il est possible d'obtenir de nouvelles teintures non toxiques, qui engendrent des nuances particulièrement bien résistantes à la fois aux shampooings, à la lumière et à la transpiration, en associant de la 2-β-hydroxyéthyl) paraphénylènediamine avec, conjointement, de la 2-méthyl résorcine et de la résorcine.

Cette découverte est à la base de la présente invention.

La présente invention a ainsi pour objet une composition de teinture d'oxydation pour fibres kératiniques, en particulier pour fibres kératiniques humaines, telles que les cheveux, du type comprenant, dans un milieu approprié pour la teinture, au moins un précurseur de colorant d'oxydation et au moins un coupleur, et qui est caractérisée par le fait qu'elle contient, à titre de précurseur de colorant d'oxydation, de la 2-β-hydroxyéthyl paraphénylènediamine, et, à titre de coupleur, une association de la 2-méthyl résorcine et de la résorcine, ou les sels d'addition de ces différents composés avec un acide.

En particulier, et comme le montreront les exemples donnés ci-après, les nouvelles teintures ainsi obtenues permettent d'aboutir, sur des cheveux qui ont été pourtant sensibilisés par des traitements de déformation permanente ou de décoloration, à des nuances bien plus résistantes à la fois aux shampooings, à la lumière et à la transpiration que celles renfermant i) de la 2-β-hydroxyéthyl paraphénylènediamine associée à de la 2-méthyl résorcine ou ii) de la 2-β-hydroxyéthyl paraphénylènediamine associée à de la résorcine.

Un autre objet de l'invention porte sur la composition prête à l'emploi, contenant les différents agents utilisés pour la teinture des fibres kératiniques définis ci-dessus et un agent oxydant.

L'invention vise également un procédé de teinture des fibres kératiniques, et en particulier des fibres kératiniques humaines telles que les cheveux, consistant à appliquer sur ces fibres au moins une composition (A) contenant, dans un milieu approprié pour la teinture, au moins un précurseur de colorant d'oxydation et au moins deux coupleurs tels qu'ils ont été définis ci-avant, la couleur étant révélée à pH alcalin, neutre ou acide à l'aide d'un agent oxydant qui est ajouté juste au moment de l'emploi à la composition (A) ou qui est présent dans une composition (B) appliquée simultanément ou séquentiellement de façon séparée.

L'invention a également pour objet des dispositifs de teinture ou "kits" à plusieurs compartiments, dont le premier

compartiment contient au moins la 2-β-hydroxyéthyl paraphénylènediamine à titre de précurseur de colorant d'oxydation, et au moins l'association de la 2-méthyl résorcine et de la résorcine à titre de coupleurs, et le deuxième compartiment un agent oxydant.

D'autres caractéristiques, aspects, objets et avantages de l'invention apparaîtront encore plus clairement à la lecture de la description et des exemples qui suivent.

Les sels d'acide qui peuvent être utilisés selon l'invention sont choisis de préférence parmi les chlorhydrates, les sulfates, les bromhydrates et les tartrates.

La concentration en précurseur de colorant d'oxydation, ou en ses sels, peut varier de préférence entre 0,01 et 10 % en poids environ par rapport au poids total de la composition de teinture, et encore plus préférentiellement entre 0,05 et 5 % en poids environ.

La concentration en 2-méthyl résorcine ou en ses sels peut varier de préférence entre 0,005 et 5 % en poids environ par rapport au poids total de la composition de teinture, et encore plus préférentiellement entre 0,05 et 3 % en poids environ.

La concentration en résorcine ou en ses sels peut varier de préférence entre 0,005 et 5 % en poids environ par rapport au poids total de la composition de teinture, et encore plus préférentiellement entre 0,05 et 3 % en poids environ.

L'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.

La composition (A), qui renferme l'association des colorants telle que décrite ci-dessus, peut avoir un pH compris entre 3 et 11, qui peut être ajusté à la valeur choisie soit au moyen d'agents alcalinisants habituellement utilisés en teinture des fibres kératiniques, tels que l'ammoniaque, les carbonates alcalins, les alcanolamines, par exemple les mono-, di- et tri- éthanolamines ainsi que leurs dérivés, les hydroxydes de sodium ou de potassium, les composés de formule :

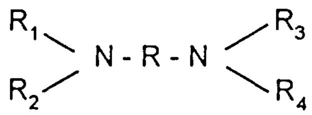

dans laquelle R est un reste propylène éventuellement substitué par un groupement hydroxyle ou un radical alkyle en $C_1$-$C_4$ ; $R_1$ $R_2$, $R_3$ et $R_4$, simultanément ou indépendamment l'un de l'autre, représentent un atome d'hydrogène, un radical alkyle en $C_1$-$C_4$ ou hydroxyalkyle en $C_1$-$C_4$,
soit au moyen d'agents acidifiants classiques, tels que les acides minéraux ou organiques comme par exemple les acides chlorhydrique, tartrique, citrique et phosphorique.

Le pH de la composition (B) renfermant l'agent oxydant tel que défini ci-dessus, est tel qu'après un mélange avec la composition (A), le pH de la composition appliquée sur les fibres kératiniques humaines varie de préférence entre 3 et 11. Il est ajusté à la valeur désirée à l'aide d'agents acidifiants ou éventuellement alcalinisants bien connus de l'état de la technique, tels que ceux décrits ci-dessus.

Comme indiqué précédemment, la composition oxydante (B) est de préférence constituée par une solution d'eau oxygénée.

Selon un mode de réalisation préféré du procédé de teinture de l'invention, on mélange, au moment de l'emploi, la composition de teinture (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on rince, on lave au shampooing, on rince à nouveau et on sèche.

Les compositions de teinture peuvent encore contenir, en plus des colorants définis ci-dessus, d'autres coupleurs et/ou des colorants directs, notamment pour modifier les nuances ou les enrichir en reflets.

Les compositions de teinture peuvent également contenir des agents antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tert.butyl hydroquinone et l'acide homogentisique et sont alors généralement présents dans des proportions comprises entre environ 0,05 et 1,5 % en poids par rapport au poids total de la composition.

Les compositions de teinture contiennent également, dans leur forme de réalisation préférée, des agents tensioactifs bien connus de la technique, dans des proportions comprises entre environ 0,5 et 55 % en poids, et de préférence entre 2 et 50 % en poids, par rapport au poids total de la composition, des solvants organiques, dans des proportions

comprises entre environ 1 et 40 % en poids, et en particulier entre 5 et 30 % en poids par rapport au poids total de la composition, ou tout autre adjuvant cosmétiquement acceptable et connu de la technique antérieure en teinture d'oxydation capillaire.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

Des exemples concrets illustrant l'invention vont maintenant être donnés. On commencera par définir les tests utilisés pour évaluer les performances des teintures d'oxydation selon l'invention, concernant leur résistance aux shampooings, à la lumière et à la transpiration.

Résistance aux shampooings (machine Ahiba-Texomat) :

On place des mèches de cheveux teints dans un panier que l'on immerge dans une solution d'un shampooing standard. Le panier est soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on retire les mèches que l'on rince puis sèche. Les mèches teintes peuvent être soumises à plusieurs épreuves shampooings consécutives.

Résistance à la lumière (Xenotest) :

Les cheveux teints sont fixés sur un support (carton ou plastique). Ces supports sont disposés sur des porte-échantillons qui tournent autour d'une lampe Xénon pendant une durée variant de 20 à 80 heures sous un taux d'humidité variant de 25 à 75 % HR (Humidité Relative) et à une température de 25° C.

Résistance à la transpiration :

On utilise une solution de sueur synthétique de composition suivante :

| | |
|---|---|
| - NaCl | 1 g |
| - Hydrogénophosphate de potassium | 0,1 g |
| - Histidine | 0,025 g |
| - Acide lactique q.s. | pH 3,2 |
| - Eau distillée q.s.p. | 100 g |

Dans un cristallisoir recouvert d'un verre de montre et renfermant cette solution de sueur, on immerge les mèches de cheveux teints qu'on laisse séjourner de 20 à 50 heures à 37° C. On rince ensuite les mèches et on les sèche.

**EXEMPLES**

**EXEMPLE 1**

On a préparé la composition de teinture, conforme à l'invention, suivante :

| | |
|---|---|
| - 2-βhydroxyéthyl paraphénylènediamine, dichlorhydrate | 0,60 g |
| - 2-méthyl résorcine | 0,20 g |
| - résorcine | 0,20 g |
| - Alcool oléique polyglycérolé à 4 moles de glycérol, à 78 % de matières actives (M.A.) | 5,7 g M.A. |
| - Alcool oléique polyglycérolé à 2 moles de glycérol | 4,0 g |
| - Acide oléique | 3,0 g |
| - Amine oléique à 2 moles d'oxyde d'éthylène, vendue sous la dénomination Ethomeen 012 par la Société AKZO | 7,0 g |
| - Laurylamino succinamate de diéthylaminopropyle, sel de sodium à 55 % de M.A. | 3,0 g M.A. |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35 % de MA | 0,46 g M.A. |
| - Acétate d'ammonium | 0,8 g |
| - Antioxydant, séquestrant, | q.s. |
| - Parfum, conservateur, | q.s. |
| - Solution aqueuse d'ammoniaque à 20 % de $NH_3$ | 2,0 g M.A. |
| - Eau déminéralisée q.s.p. | 100,0 g |

On a réalisé, parallèlement, deux compositions comparatives qui renfermaient, en remplacement de l'association colorante ternaire selon l'invention constituée de la 2-β-hydroxyéthyl paraphénylènediamine, dichlorhydrate + 2-méthyl résorcine + résorcine, les associations colorantes binaires suivantes :

*<u>Composition comparative (A)</u>* :

2-β-hydroxyéthyl paraphénylènediamine, dichlorhydrate (0,6g) + 2-méthyl résorcine (0,4 g),

*<u>Composition comparative (B)</u>* :

2-β-hydroxyéthyl paraphénylènediamine, dichlorhydrate (0,6 g) + résorcine (0,4g).

Au moment de l'emploi, on a mélangé chacune de ces trois compositions, poids pour poids, avec de l'eau oxygénée titrant 20 volumes (6% en poids), de pH 3.

On a obtenu trois mélanges de pH 9,8.

On a appliqué ensuite ces mélanges sur les cheveux à teindre pendant 30 minutes ; puis on a rincé les cheveux, on les a lavés au shampooing, on les a rincés à nouveau et enfin on les a séchés.

***Evaluation de la résistance aux shampooings :***

Après avoir teint des cheveux de même nature (gris permanentés à 90 % de blancs) avec respectivement chacune des trois compositions ci-dessus : la composition de l'invention et les deux compositions comparatives (A) et (B), on a soumis les cheveux teints au test de résistance aux shampooings (3 shampooings) décrit ci-avant.

On a exprimé la dégradation de la couleur entre les cheveux teints et ceux teints ayant subi trois épreuves shampooing, au moyen de l'équation de NICKERSON qui définit les indices de variation de couleur :

$$\Delta E = 0{,}4\ C_0\ \Delta H + 6\Delta V + 3\Delta C\ ;$$

cette équation est décrite dans la publication : "Journal of the Optical Society of America ", 1944 - sept - Vol. 34 - n°9 - p 550-570, les paramètres H, V et C représentant les paramètres de la notation MUNSELL (Norme ASTM D 1535-68), laquelle définit la couleur, (H désignant la nuance ou HUE, V désignant l'intensité ou VALUE, C désignant la pureté ou CHROMATICITE et $C_0$ désignant la pureté de la mèche par rapport à laquelle on désire évaluer la différence de couleur).

Les dégradations de la couleur enregistrées avec la composition selon l'invention et celles des compositions comparatives (A) et (B) sont réunies dans le tableau I ci-dessous :

Tableau I

| Composition de teinture | Variation de couleur entre cheveux teints et cheveux teints ayant subi trois épreuves shampooing (ΔE) |
|---|---|
| Composition de l'invention | 1,48 |
| Composition comparative (A) | 4,15 |
| Composition comparative (B) | 3,25 |

La dégradation de couleur (ΔE) étant d'autant plus importante que le chiffre indiqué est élevé, on constate donc bien, de façon inattendue et surprenante, une résistance aux shampooings bien supérieure avec la composition de teinture selon l'invention.

***Evaluation de la résistance à la lumière :***

Après avoir teint des cheveux de même nature (châtain décolorés) avec respectivement chacune des trois compositions ci-dessus : la composition de l'invention et les deux compositions comparatives (A) et (B), on a soumis les cheveux teints au test de résistance à la lumière décrit ci-avant.

On a exprimé la dégradation de la couleur entre les cheveux teints et ceux teints ayant subi une épreuve lumière de 40 heures, au moyen de l'équation de NICKERSON décrite ci-dessus.

Les dégradations de la couleur enregistrées avec la composition selon l'invention et celles des compositions comparatives (A) et (B) sont réunies dans le tableau II ci-dessous :

Tableau II

| Composition de teinture | Variation de couleur entre cheveux teints et cheveux teints ayant subi une épreuve lumière de 40h (ΔE) |
|---|---|
| Composition de l'invention | 1,48 |
| Composition comparative (A) | 2,86 |
| Composition comparative (B) | 2,74 |

La dégradation de couleur (ΔE) étant d'autant plus importante que le chiffre indiqué est élevé, on constate donc bien, de façon inattendue et surprenante, une résistance à la lumière nettement supérieure avec la composition de teinture selon l'invention.

***Evaluation de la résistance à la transpiration :***

Après avoir teint des cheveux de même nature (châtain décolorés) avec respectivement chacune des trois compositions ci-dessus : la composition de l'invention et les deux compositions comparatives (A) et (B), on a soumis les cheveux teints au test de résistance à la transpiration décrit ci-avant (temps de contact : 48 heures).

On a exprimé la dégradation de la couleur entre les cheveux teints et ceux teints ayant subi une épreuve à la transpiration, au moyen de l'équation de NICKERSON décrite ci-dessus.

Les dégradations de la couleur enregistrées avec la composition selon l'invention et celles des compositions comparatives (A) et (B) sont réunies dans le tableau III ci-dessous :

Tableau III

| Composition de teinture | Variation de couleur entre cheveux teints et cheveux teints ayant subi une épreuve à la transpiration (ΔE) |
|---|---|
| Composition de l'invention | 1,74 |
| Composition comparative (A) | 3,30 |
| Composition comparative (B) | 4,96 |

La dégradation de couleur (ΔE) étant d'autant plus importante que le chiffre indiqué est élevé, on constate donc bien, de façon inattendue et surprenante, une résistance à la transpiration bien supérieure avec la composition de teinture selon l'invention.

**Revendications**

1. Composition de teinture d'oxydation pour fibres kératiniques, en particulier pour fibres kératiniques humaines telles que les cheveux, du type comprenant, dans un milieu approprié pour la teinture, au moins un précurseur de colorant d'oxydation et au moins un coupleur, caractérisée par le fait qu'elle contient, à titre de précurseur de colorant d'oxydation, de la 2-β-hydroxyéthyl paraphénylènediamine et, à titre de coupleur, une association de 2-méthyl résorcine et de résorcine, ou les sels d'addition de ces différents composés avec un acide.

2. Composition de teinture selon la revendication 1, caractérisée par le fait que les sels d'addition avec un acide sont choisis parmi les chlorhydrates, les sulfates, les bromhydrates et les tartrates.

3. Composition de teinture selon l'une des revendications 1 à 2, caractérisée par le fait que la 2-β-hydroxyéthyl paraphénylènediamine ou ses sels est présente dans une concentration comprise entre 0,01 et 10 % en poids par rapport au poids total de la composition, de préférence entre 0,05 et 5 % en poids, que la 2-méthyl résorcine ou ses sels est présente dans une concentration comprise entre 0,01 et 5 % en poids par rapport au poids total de la composition, de préférence entre 0,05 et 3 % en poids, et que la résorcine ou ses sels est présente dans une concentration comprise entre 0,01 et 5% en poids par rapport au poids total de la composition, de préférence entre 0,05 et 3% en poids.

4. Composition de teinture selon l'une quelconque des revendications 1 à 3, prête à l'emploi, caractérisée en ce qu'elle contient en outre un agent oxydant et qu'elle possède un pH compris entre 3 et 11.

5. Procédé de teinture des fibres kératiniques et en particulier des fibres kératiniques humaines telles que les cheveux, caractérisé par le fait qu'il consiste à appliquer sur les fibres une composition de teinture (A) selon l'une quelconque des revendications 1 à 3, et à révéler la couleur en milieu alcalin, neutre ou acide à l'aide d'un agent oxydant qui est ajouté juste au moment de l'emploi à cette composition (A) ou qui est présent dans une composition (B) appliquée simultanément ou séquentiellement de façon séparée.

6. Dispositif à plusieurs compartiments ou "Kit" pour la teinture des fibres kératiniques et en particulier des fibres kératiniques humaines telles que les cheveux, caractérisé par le fait qu'il comporte au moins deux compartiments, dont l'un d'entre eux renferme une composition (A) telle que définie dans les revendications 1 à 3, et un autre une composition (B) comprenant un agent oxydant dans un milieu approprié pour la teinture.

7. Utilisation d'une composition de teinture selon l'une quelconque des revendications 1 à 4 ou d'un dispositif de teinture ou "Kit" à plusieurs compartiments selon la revendication 6, pour la teinture des fibres kératiniques humaines telles que les cheveux.

## Claims

1. Oxidation dyeing composition for keratinous fibres, in particular for human keratinous fibres such as hair, of the type comprising, in a medium appropriate for dyeing, at least one oxidation dye precursor and at least one coupler, characterized in that it contains, as oxidation dye precursor, 2-(β-hydroxyethyl)-paraphenylenediamine and, as coupler, a combination of 2-methylresorcinol and resorcinol, or the addition salts of these various compounds with an acid.

2. Dyeing composition according to Claim 1, characterized in that the addition salts with an acid are chosen from hydrochlorides, sulphates, hydrobromides and tartrates.

3. Dyeing composition according to either of Claims 1 and 2, characterized in that 2-(β-hydroxyethyl)-paraphenylenediamine or its salts is present in a concentration of between 0.01 and 10 % by weight with respect to the total weight of the composition and preferably between 0.05 and 5 % by weight, in that 2-methylresorcinol or its salts is present in a concentration of between 0.01 and 5 % by weight with respect to the total weight of the composition and preferably between 0.05 and 3 % by weight and in that resorcinol or its salts is present in a concentration of between 0.01 and 5 % by weight with respect to the total weight of the composition and preferably between 0.05 and 3 % by weight.

4. Dyeing composition according to any one of Claims 1 to 3, which is ready-to-use, characterized in that it additionally contains an oxidizing agent and in that it has a pH of between 3 and 11.

5. Process for dyeing keratinous fibres and in particular human keratinous fibres such as hair, characterized in that it comprises applying to the fibres a dyeing composition (A) according to any one of Claims 1 to 3 and in developing the colour in alkaline, neutral or acidic medium using an oxidizing agent which is added to this composition (A) only at the time of use or which is present in a composition (B) separately applied simultaneously or sequentially.

6. Multi-compartment device or "kit" for dyeing keratinous fibres and in particular human keratinous fibres such as hair, characterized in that it contains at least two compartments, one of which contains a composition (A) such as defined in Claims 1 to 3 and another of which contains a composition (B) comprising an oxidizing agent in a medium appropriate for dyeing.

7. Use of a dyeing composition according to any one of Claims 1 to 4 or of a multi-compartment dyeing device or "kit" according to Claim 6 for dyeing human keratinous fibres such as hair.

## Patentansprüche

1. Zusammensetzung zum oxidativen Färben von Keratinfasern und insbesondere menschlichen Keratinfasern, wie dem Haar, die in einem zum Färben geeigneten Medium mindestens ein Farbstoffvorprodukt eines Oxidationsfarbstoffs und mindestens einen Kuppler enthält, dadurch gekennzeichnet, daß sie als Farbstoffvorprodukt des Oxidationsfarbstoffs 2-(β-Hydroxyethyl)-p-phenylendiamin und als Kuppler eine Kombination von 2-Methylresorcin und Resorcin oder die Additionssalze dieser verschiedenen Verbindungen mit einer Säure enthält.

2. Zusammensetzung zum Färben nach Anspruch 1, dadurch gekennzeichnet, daß die Additionssalze mit einer Säure unter den Hydrochloriden, den Sulfaten, den Hydrobromiden und den Tartraten ausgewählt sind.

3. Zusammensetzung zum Färben nach Anspruch 1 oder 2, dadurch gekennzeichnet, daß das 2-(β-Hydroxyethyl)-p-phenylendiamin oder seine Salze in einer Konzentration im Bereich von 0,01 bis 10 Gew.-% und vorzugsweise im Bereich von 0,05 bis 5 Gew.-% vorliegen, bezogen auf das Gesamtgewicht der Zusammensetzung, daß das 2-Methylresorcin oder seine Salze in einer Konzentration im Bereich von 0,01 bis 5 Gew.-% und vorzugsweise im Bereich von 0,05 bis 3 Gew.-% vorliegen, bezogen auf das Gesämtgewicht der Zusammensetzung, und daß das Resorcin oder seine Salze in einer Konzentration im Bereich von 0,01 bis 5 Gew.-% und vorzugsweise im Bereich von 0,05 bis 3 Gew.-% vorliegen, bezogen auf das Gesamtgewicht der Zusammensetzung.

4. Gebrauchsfertige Zusammensetzung zum Färben nach einem der Ansprüche 1 bis 3, dadurch gekennzeichnet, daß sie ferner ein Oxidationsmittel enthält und daß sie einen pH-Wert im Bereich von 3 bis 11 aufweist.

5. Verfahren zum Färben von Keratinfasern und insbesondere menschlichen Keratinfasern, wie dem Haar, dadurch gekennzeichnet, daß es darin besteht, auf die Fasern eine Färbemittelzusammensetzung (A) nach einem der

Ansprüche 1 bis 3 aufzubringen und in einem alkalischen, neutralen oder sauren Medium mit einem Oxidationsmittel, das unmittelbar bei der Anwendung zu dieser Zusammensetzung (A) gegeben wird oder das in einer Zusammensetzung (B) vorliegt, die gleichzeitig oder nachfolgend getrennt davon aufgebracht wird, die Farbe zu entwickeln.

6. Vorrichtung mit mehreren Abteilungen oder 'Kit' für das Färben von Keratinfasern und insbesondere menschlichen Keratinfasern, wie dem Haar, dadurch gekennzeichnet, daß sie mindestens zwei Abteilungen umfaßt, wobei eine davon eine wie in den Ansprüchen 1 bis 3 definierte Zusammensetzung (A) und die andere eine Zusammensetzung (B) enthält, die in einem zum Färben geeigneten Medium ein Oxidationsmittel enthält.

7. Verwendung einer Färbemittelzusammensetzung nach einem der Ansprüche 1 bis 4 oder einer Vorrichtung zum Färben oder eines 'Kits' mit mehreren Abteilungen nach Anspruch 6 zum Färben von menschlichen Keratinfasern wie dem Haar.